# EUROPEAN PATENT APPLICATION

(11) **EP 4 342 456 A2**
(43) Date of publication of application: **27.03.2024**
(21) Application number: 24157415.1
(22) Date of filing: 03.04.2009
(51) Int. Cl.: A61K 9/00

(54) **PLANT FIBER PRODUCT AND METHOD FOR ITS MANUFACTURE**

(62) Divisional of application: 22189511.3
(71) Applicant: British American Tobacco Sweden AB, 215 32 Malmö (SE)
(72) Inventor: BJORKHOLM, Lars, 2900 Hellerup (DK); HUBINETTE, Fredrik, 752 67 Uppsala (SE)
(74) Representative: Gill, Siân Victoria

(57) **Abstract**

The invention relates to a product for oral use, said product containing a mixture of at least: cellulose fibers; an alginate composition; and at least one added substance intended to be released from the product when said product is used. The alginate composition is dispersed in the product and the alginate composition comprises at least water, alginate and the added substance, said composition containing an alginate matrix that firmly retains at least a major proportion of the added substance so long as the matrix is intact, and the alginate matrix being formed so as to disintegrate and/or dissolve in the chemical and physical environment that exists in a user's mouth. The invention also relates to a method for preparing such a product.

## Description

### TECHNICAL FIELD

The invention relates to a plant fiber product and also a method for producing such a product. In particular, the invention relates to regulating the rate of release of substances, for example caffeine, nicotine and other active substances, added to the product.

### BACKGROUND ART

There are a number of different plant fiber products intended to impart a taste when used orally and to deliver substances, for example nicotine and caffeine, to the user via the mucous membranes of the mouth. Normally such products include tobacco, for example snuff (dry or moist) and chewing tobacco, although a number of tobacco-free alternatives in which the product is based on other plant fibers are known, for example from SE 529 886 (buckwheat and maize fibers), US 2007/0000506 (coffee and tea) and WO 2009/010884 (tea and citrus fibers).

It is known, for example from WO 2009/010884, to add functional or active substances, for example caffeine, mint, cinnamon, antioxidants and vitamins, to such oral products.

When using products of this type, added substances have a tendency to dissolve relatively quickly and become available through the mucous membranes for absorption in the blood stream of the user. Such a rapid rate of delivery can be advantageous in certain situations, but sometimes a slower delivery is better, with the added substances being delivered to the user at a slower delivery rate but over a longer period, possibly within one hour.

In order to prolong and be able to control the rate of delivery, it has been proposed in WO 2009/010884 to use small, solid capsules that can contain taste substances and other substances and that can, for example, be crushed by the user's teeth when so desired. Exactly how these capsules are to be formulated or produced is not disclosed however.

There is therefore still a need for solutions to the problem of regulating the rate of release of added substances.

### DISCLOSURE OF INVENTION

The object of this invention is to provide a plant fiber product for oral use that permits better regulation of the rate of release of added substances than is achieved in existing products. This object is achieved by a product and a method as claimed in claims 1 and 11. The dependent claims disclose advantageous embodiments, developments and variants of the invention.

The invention relates to a plant fiber product for oral use, said product containing a mixture of at least plant fibers and at least one added substance that is intended to be released from the product when said product is used.

The product according to the invention contains an alginate composition distributed in the product and comprising at least water, alginate and the added substance, said composition containing an alginate matrix that retains at least a major proportion of the added substance so long as the matrix is intact, and the alginate matrix being formed so as to disintegrate and/or dissolve in the chemical and physical environment that exists in a user's mouth.

An advantageous effect of such a product is that the alginate matrix gradually disintegrates and/or dissolves when the product is used, which means that the added substance is gradually released in the mouth. In this way the release of the actual substance is regulated so that it takes place over a certain period of time. In the case where the added substance is an active substance intended to be absorbed by the user, for example caffeine or nicotine, a favorable delivery of the active substance from the product to the bloodstream of the user through the mucous membranes can be achieved so long as the matrix continues to dissolve/disintegrate. In the case where the added substance consists of a taste substance, said taste substance is delivered over a prolonged period to the user's oral cavity, which means that the taste sensation can be maintained for a longer time. By varying, for example, the type of alginate(s) or its/their relative amount in the composition, the alginate matrix can be formulated or prepared insuch a way that the rate of delivery of the added substance is modified and is even made to vary over time.

A further advantageous effect of the product according to the invention is that until the release of the bound, added substance, the alginate composition/alginate matrix is present as a protective covering over the added substance or substances. This means, for example, that oxidation- sensitive substances are protected from coming into contact with oxidizing agents. Such oxidizing agents can, for example, originate from the plant fibers or can be added during the (pre)treatment of the latter. The protective covering means that it is also possible to maintain a suitable pH in the local environment of the added substances. The pH can also be affected by substances originating from the plant fibers or from their treatment. Antioxidants and/or pH buffer substances, for example, can be included in the alginate composition according to the invention so as to provide further protection of active substances within the protective covering.

Alginate, the salt of alginic acid, is a well-known substance that is commercially available. It is used, for example, as a thickener and gelling agent in the production of foodstuffs and similar products, for example plant fiber products of the type concerned in the present specification.

The expression "oral use" refers to the product, in normal use, being placed somewhere in the oral cavity of the user, for example under the lips, in the same way as moist snuff products are generally used.

The expression "added substance that is intended to be released from the product when said product is used" denotes a substance or an agent that is added to the product in addition to all the substances that may originate from the plant fibers. Plant fibers that are suitable for the product according to the invention may contain substances such as theine, caffeine or nicotine that are also released when the product is used. The expression is also understood to denote a substance that is specifically intended to be released from the product when used with the object of imparting a taste, to be swallowed or absorbed by the user through the mucous membranes of the mouth.

The expression "alginate matrix" denotes the network that an alginate forms when it is dissolved in water. An alginate matrix is a form of solution/mixture whose viscosity and readiness to dissolve generally vary with, for example, the type of alginate, ratio of alginate/water, temperature, etc.

In a preferred embodiment of the invention, the product is adapted to deliver substances to a user through mucous membranes in the user's mouth and, in addition, said at least one added substance is an active substance, for example caffeine, that can be absorbed through the mucous membranes in the mouth when the product is used.

The expression "active substance that can be absorbed through the mucous membranes in the mouth" denotes, firstly, those substances that permit delivery to the user's bloodstream through the oral mucous membranes. Secondly, the term "active substance" is understood to mean those substances which, after they have been absorbed, have a significant effect on the user, for example substances that have a stimulating action on the central nervous system, have an energizing effect, an antipyretic or analgesic action, or substances that are useful for the body, for example vitamins, antioxidants and minerals.

In a preferred embodiment of the invention, the at least one active substance comprises one or more substances belonging to any of the following categories: API (active pharmaceutical substances), food additives, natural medicaments or naturally occurring substances that can have an effect on humans. Examples of such substances are caffeine, nicotine, vitamin B12, vitamin C, vitamin E, bioperin, Q10, selenium, glutathione, liponic acid, folic acid, ginseng, pollen extract, antioxidants, minerals, paracetamol, acetylsalicylic acid, Russian root and rose root, etc.

In another preferred embodiment of the invention, said at least one added substance is a taste substance.

In another preferred embodiment of the invention, said alginate includes an alginate salt of monovalent cations. Preferably, said alginate is soluble in cold water. Such an alginate allows the formation of an alginate matrix having suitable stability and also allows a simple production process.

In another preferred embodiment of the invention, said alginate includes at least two different types of alginate with different dissolution or decomposition properties. Typically the different types of alginate have different viscosities in the dissolved state. Such a combination of alginates can be used to form an alginate matrix that exhibits a special release curve in which, for example, a less viscous alginate permits a relatively high initial release, whereas a more viscous alginate allows the release to proceed more or less continuously for a longer time. The alginate matrix is therefore in this case composed of two or more types of alginates.

In another preferred embodiment of the invention, the plant fibers are based on one or more of the following plants: tea, coffee, tobacco, cocoa, maize, herbs, yerba mate and cellulose. Tea fibers preferably make up a significant proportion. In addition the plant fibers preferably exhibit a size distribution, for example after grinding and screening, principally in the range 0.1 - 0.9 mm, and the plant fibers are preferably steam treated. Such a treatment of the plant fibers produces an advantageous product that can for example be baked.

The invention also relates to a method for preparing a plant fiber product for oral use, comprising the following steps: mixing plant fibers and other components included in the product. The method according to the invention is characterized in that it also includes the following steps: (i) admixing an alginate composition containing at least water, alginate and at least one added substance intended to be released from the product when said product is used, the composition at least in part forming an alginate matrix that retains at least a major proportion of the added substance so long as the matrix is intact, and the alginate matrix being formed so as to disintegrate and/or dissolve in the chemical and physical environment that exists in a user's mouth, and (ii) mixing plant fibers with said alginate composition.

### PREFERRED EMBODIMENTS

The product according to the invention typically contains water, treated plants fibers and one or more added substances in the form of taste substances and one or more added active substances, for example caffeine. Apart from the added active substances, the product can of course contain active substances that originate from the plant fiber. The product can also include colorants.

The plant fibers normally form a major component of the product and, among other things, give the product its structure. It is well known that plant fibers can be treated in many different ways for the type of products concerned here.

In the preferred example that is described here, the plant fibers consist of tea fibers. These are suitably (pre)treated similarly to the way moist snuff is produced. The main features of this treatment can be described by the following method steps:
A) Drying, grinding and screening. A suitable particle size and size distribution for the tea fibers is in principle 0.1 - 0.9 mm with an average value around 0.5 mm.
B) Addition of water and salt.
C) Heating, injection of steam, and mixing.
D) Cooling.
E) Addition of, for example, sodium carbonate, preservatives, taste substances, humectants and binders.

Tea fibers treated in this way provide a bakeable product that can be pressed into portions like moist snuff in loose form.

Step E is preferably followed by an intermediate storage stage and a step in which a finished product is formed. This final step includes suitable packing of the product in boxes either in loose form or in the form of portion-size sachets, like pinches of snuff. Another suitable form of the end product are pressed lozenges/tablets which are suitably somewhat softened so that they can be consumed more easily when used. The finished product can also have a film-like form.

The finished plant fiber product suitably has a water content of ca. 40 - 60%, preferably 45 - 48% for a product in portion-size sachets, and 50 - 54% for a bakeable, loose product.

An example of a formulation for preferred embodiments of the product according to the invention as well as an example of a preferred method for its production are given hereinbelow.

The alginate composition that is produced according to stages 1 - 2 hereinafter is suitably added in step B and/or in step E in the process for treating the plant fibers. Heat-sensitive substances are suitably added in step E.

### Formulation:

10 parts by weight of (pretreated) tea fibers 10 parts by weight of water
2 parts by weight of Protanal^{®} LFR 5/60 (FMC BioPolymer) alginate 0.1 - 0.5 part by weight of glycerol ca. 0.3 part by weight of caffeine ca. 0.5 part by weight of vitamin B12 bergamot (in an amount according to the desired taste intensity)

In another preferred embodiment of the invention, caffeine, vitamin B12 and bergamot are omitted and instead ca. 0.01 part by weight of nicotine and liquorice root (in an amount according the desired taste intensity) is included. In another preferred embodiment of the invention, 0.5 part by weight of acetylsalicylic acid and peppermint (in an amount depending on the desired taste intensity) is included instead. It is also possible to combine the various embodiments, omit certain taste substances or active substances, and/or create variants with other active substances and taste substances.

The relative amounts of ingredients in the product can of course be varied. For example the amounts of active added substances can be adapted according to the total weight of a portion size for the actual product so that it contains suitable portion amounts. The total weight of a typical plant fiber product is around 1 - 2 g. In general, suitable portion amounts for any added substances are as follows: caffeine ca. 50 mg; vitamin B12 ca. 10 - 100 mg; nicotine ca. 1 - 3 mg; and acetylsalicylic acid ca. 100 mg.

In order that the alginate matrix can exist in a stable manner, the total amount of added substance should normally not exceed about 75% of the weight of alginate, and preferably should not exceed 50% of the weight of alginate. Depending for example on the type of alginate and the type of substance, this may mean that active substances should be added in smaller amounts than those corresponding to said generally suitable portion amounts.

### Method:

1. Preparation of an aqueous solution containing water and glycerol, active substances and taste substances (according to the above formulation).
2. Addition of alginate to the aqueous solution. Mixing so as to obtain a homogeneous (uniformly mixed) aqueous solution/alginate composition.
3. Addition of alginate composition to pretreated tea fibers.
4. Mixing to obtain a well-mixed plant fiber product.

In step 1 the object is to dissolve the components. A pH adjustment may be necessary so that certain substances, for example nicotine and paracetamol, will dissolve thoroughly. A pH adjustment may also be important in order to promote the absorption of certain active substances by the mucous membranes. Glycerol has inter alia the property of increasing the solubility but also improving the consistency of the finished product.

In step 2 it is important to add the alginate relatively slowly and to stir the mixture fairly vigorously so that the alginate is properly dissolved. Stirring for up to possibly 30 - 60 min may be necessary. An increase in temperature to around 500 C can be employed in order to accelerate the dissolution of the alginate, provided that the added active substances are not temperature sensitive. In general it is best to operate at room temperature.

Alginate generally consists of polysaccharide molecules of different lengths and type and with different types of cations that compensate the negative groups of the polysaccharide molecules. In the above example, Protanal 5/60 is used, which is a low - viscosity sodium alginate soluble in cold water. The expression soluble in cold water means that the alginate completely dissolves in water at room temperature.

When alginate is dissolved in water a matrix is formed, that is to say a form of network in which alginate molecules bind to water molecules as well as to one another, whereby the viscosity of the solution increases. The substances that are dissolved in the water, for example an added active substance, are prevented by inter alia electrostatic forces and steric hindrance from leaving the matrix and are thereby firmly retained.The viscosity that is produced when the alginate is dissolved depends mainly on the type or types of alginate (for example average molecular length and proportion of so-called G (guluronate) and M (mannuronate) blocks), concentration of alginate, and temperature.

The viscosity of the alginate composition is a good measure of the ability of the alginate matrix to firmly retain bound substances: the higher the viscosity, the more securely are the substances normally bound.

The composition that is formed when alginate is dissolved in water can be said to be a form of solution, although since the viscosity can be relatively high the composition does not necessarily exhibit those properties that are normally associated with solutions, for example the ability to be poured like a liquid.

After the above step 2, the viscosity of the solution/composition is sufficiently high for substances to be firmly bound in the alginate matrix (and thereby in the plant fiber product when the composition is distributed therein) under normal conditions, that is to say before the product is used and when, for example, no further substances are added that can break up or dissolve the matrix. At the same time the viscosity in this example is sufficiently low so as to allow the alginate composition to be pumped in the production process and enable a simple and effective mixing with the plant fibers. Furthermore the viscosity of the composition is sufficiently low so as to enable the bound substances to be released at a suitable rate from the matrix when the product is used (see below). At higher viscosity, the solution/matrix may have to be mechanically broken up so that it can be thoroughly distributed during the mixing with the plant fibers. Too high a viscosity can prevent or to a very large extent delay the release of certain bound substances when the product is used.

On account of the fact that the active substances in step 1 are added and distributed in an aqueous solution, and since this solution (even if in a more viscous form) is mixed in a later step with plant fibers and is thereby distributed in the product, the active substances, despite the fact that they represent only a relatively very small proportion, can be homogeneously distributed in the finished plant fiber product. A good distribution of smallamounts of active substance in dry form is significantly more difficult to achieve.

In stage 3 the alginate composition is combined with the tea fibers so that in stage 4 a thoroughly mixed product mixture is formed. The composition containing the alginate matrix and the substances bound therein is thereby distributed in the plant fiber product in the form of a layer of the fibers and/or in the form of smaller, isolated units ("droplets").

So long as the plant fiber product is not subjected to any special action, for example evaporation, elevated temperature, or the addition of a large amount of water or other liquid, the bound substances are firmly retained in the alginate matrix. Provided that the product is kept in a suitable manner, for example airtight, under cool conditions and protected against sunlight, the bound substances can be firmly retained in the matrix for possibly up to 1 - 3 years.

When the product is placed in the mouth by a user, for example under the lips in the way moist snuff is used, the conditions are now completely different since the chemical and physical environment that exists in a user's mouth causes the alginate matrix to break down and/or dissolve. In particular this is due to the presence of saliva, which on account of its water content acts like an increased amount of solvent that dissolves the matrix and furthermore contains amylase that breaks down the polysaccharides of the alginate matrix into smaller, more easily dissolved constituents. In addition, the relatively high temperature in the mouth, compared for example to a cool storage location, contributes to the disintegration and dissolution of the matrix

In this way the matrix is progressively dissolved and/or broken down, which over time causes the release of the previously bound substances. Active substances that are no longer bound in the matrix come into contact, largely through the saliva, with the mucous membranes in the mouth and in this way pass into the user's bloodstream. In this way, the active substances are also absorbed over a period of time. As regards taste substances that are released from the matrix, these pass into the mouth and produce the taste sensation. In this way, the product imparts a taste for a longer period of time than if the taste substances were not bound in the matrix.

By adapting the alginate composition, that is to say above all the alginate matrix, the total release time can be adapted and the release curve of the added substance or substances is given a suitable form. An important parameter is the viscosity, which for example can be adapted by varying the amount of alginate in relation to the amount of water in the composition, by varying the type of alginate, and by varying the relative amounts of different alginates. The alginate composition can also be adapted after it has been added and mixed with the plant fibers, for example by subjecting the plant fiber product to evaporation. Evaporation leads to a reduced water content, which for example affects the viscosity of the alginate matrix.

The treated plant fibers can contain substances that affect the alginate composition. For example, certain substances can have a special effect on the alginate matrix. Furthermore, for example, the pH and water content can vary in the plant fiber mixture, depending on the type of fibers and the treatment process, which can affect the alginate composition. The alginate composition is suitably adapted to the specific situation.

The release time for firmly retained substances depends inter alia also on the size of the product that is placed in the mouth, since a larger product leads, for example, to longer diffusion times. For a typical user and a typical size of the product, that is to say roughly the same as a normal pinch of snuff, according to the described embodiment of the invention, a total release time of ca. 30 - 60 minutes is obtained, that is to say the product delivers active substance and/or taste substance to the user during ca. 30 - 60 minutes from the time the product was placed in the mouth.

It goes without saying that the plant product according to the invention can of course contain additional amounts of water, alginate and substances over and above those that are included in the particular alginate composition. It may be noted here that alginate is particularly suitable for inclusion in the plant fiber product since alginate has a healing action on mucous membranes and in addition makes a positive contribution to the baking ability of the product. In addition, alginate is suitable for imparting a softer exterior to products in the form of lozenges/tablets. Alginate that has a curative effect, promotes the baking ability and/or acts as a softener canbe derived from the alginate composition and/or be derived from further added alginate.

Examples of active substances that are suitable for addition to the product by binding to an alginate matrix as described above are preferably those that belong to any of the following categories: API (active pharmaceutical substances), food additives, natural medicaments and naturally occurring substances that can have an effect on humans (also placebo effect). Specific examples of such substances are caffeine, nicotine, vitamin B12, vitamin C, vitamin E, bioperin, Q10, selenium, glutathione, liponic acid, folic acid, ginseng, pollen extract, antioxidants, minerals, paracetamol, acetylsalicylic acid, Russian root and rose root, etc. Accordingly, active substances are understood to mean those substances that have a significant (perceivable) effect on the user, for example substances that act as central nervous stimulants, have an energizing effect, antipyretics or analgesics, or substances that are useful for the body, for example vitamins, antioxidants and minerals.

Examples of taste substances that are suitable for addition to the product through binding to an alginate matrix as described above are liquorice, mint, bergamot, aniseed, citrus compounds, elder, etc.

As regards the viscosity of the alginate composition, it can be said in general that, with increasing viscosity, the ability to form hard, sparingly soluble compounds/mixtures also increases. A lower viscosity means a quicker wetting of the mixture as well as a quicker dissolution. More liquid is required to dissolve a highly viscous mixture since the ability to bind and retain water is a function of the viscosity. The proportion of G and M blocks also has an effect on the dissolution/decomposition time. A higher proportion of G block accelerates the dissolution as well as adhesion to the mucous membranes. Longer polysaccharides produce a higher viscosity and more sparingly soluble mixtures. These also require more saliva for their dissolution. Shorter polysaccharides produce a lower viscosity and are easier to dissolve with a smaller amount of saliva.

The polysaccharides are in the form of a salt having a positive charge. Furthermore, there are partial charges and functional groups/sites along the polysaccharide that can cause substances to bind to these functional sites via ionic bonds, van der Waal's forces and dipole bonds. In addition,the purely mechanical bonding between the polysaccharides means that substances are firmly held in the matrix.

The mechanical function also contributes to the viscosity of the alginate composition. Longer steric molecules attach more easily to one another and are firmly held by mechanical and chemical bonds together with other solvents and incorporated substances.

The invention is not restricted to the embodiments described above but can be varied within the scope of the invention that is defined by the following claims. As an example, it is not necessary that the plant fiber that is used is based on tea. Besides mixing different types of tea, it is also possible to base the plant fibers on for example herbs, cocoa, coffee, yerba mate, maize, cellulose and liquorice root. Tobacco can also be used. It is also possible to use a mixture of plant fibers from two or more different plants.

The described method for tea fibers is also suitable for other plant fibers.

As an additional feature of the invention, active substances and/or taste substances can be added to the plant fiber product in a conventional manner, that is to say without being bound to any alginate matrix.

Suitable alginates for the invention are in general alginate salts of monovalent cations that are soluble in cold water and have a low viscosity. An alternative to the alginate LFR 5/60 exemplified above is Protanal^{®} LF 10/60 (FMC BioPolymer), which is also a sodium alginate salt but with a higher viscosity. The higher viscosity requires a longer time for dissolution and also results in a longer total time and release time of ca. 1 - 1.5 hours. In order to dissolve LF 10/60, normally a larger amount of water per part by weight of alginate is required than is used in the above formulation

Other mixtures of alginate salts with other dissolution or decomposition properties can be utilized in order to modify the release curve of added substances. For example, two parts by weight of LFR 5/60 can be mixed with one part by weight of LF 10/60 and this alginate mixture can be incorporated into the alginate composition. With these proportions in the alginate matrix, a release profile is obtained that exhibits a relatively quick initial release, mainly due to the dissolution of the more readily dissolved LFR 5/60, which gradually changes to a more steady-state release over a longer time, mainly due to a slower dissolution of LF 10/60. By varying the proportions of LFR 5/60 and LF 10/60, the release profile or release curve can be modified further. Of course, one or more other alginates can be admixed to further adapt the dissolution/release. Release profiles and release times understandably in general depend to a large extent on the user's saliva production, how the product is kept in the mouth, and what added substance is involved.

Alginate salts of divalent cations, for example Ca, generally form irreversible solutions/matrices/gels that typically release bound substances significantly more slowly but in which certain substances can be released by diffusion at a sufficiently high rate. Also, alginate salts of divalent cations can be used in the product according to the invention, at least a relatively small amounts and in combination with one or more alginate salts of monovalent cations.

In a further method step the alginate composition can be heated in order to sterilize it before it is added to the plant fibers.

It should be noted that absorption into the blood through the mucous membranes in the mouth for example, compared to absorption through the stomach/intestines/liver, occurs more quickly and is significantly more effective, at least as regards API. This means that, in the case of absorption by the mucous membranes, the API dosage can be reduced by up to 80% depending on the liver metabolism. Such a lower dose produces fewer side effects.

### CLAUSES

The following numbered clauses form part of the description and are not part of the claims.
1. A plant fiber product for oral use, said product containing a mixture of at least:
   - plant fibers; and
   - at least one added substance intended to be released from the product when said product is used, characterized in that the product contains an alginate composition, distributed in the product and comprising at least water, alginate and the added substance, said composition containing an alginate matrix that retains at least a major proportion of the added substance so long as the matrix is intact, and the alginate matrix being formed so as to disintegrate and/or dissolve in the chemical and physical environment that exists in a user's mouth.
2. The plant fiber product according to clause 1, characterized in that the alginate composition is prepared in such a way that the alginate matrix is generated in an aqueous solution, in which solution the at least one added substance is dissolved.
3. The plant fiber product according to clause 1 or 2, characterized in that the product is adapted to deliver substances to a user through mucous membranes in the user's mouth and that said at least one added substance is an active substance, for example caffeine, that can be absorbed through the mucous membranes in the mouth when the product is used.
4. The plant fiber product according to clause 3, characterized in that the at least one active substance is one or more substances belonging to any of the following categories: API (active pharmaceutical substances), food additives, natural medicaments and naturally occurring substances that can have an effect on the human body.
5. The plant fiber product according to in any one of the preceding clauses, characterized in that said at least one added substance is a taste substance.
6. The plant fiber product according to any one of the preceding clauses, characterized in that said alginate contains an alginate salt of monovalent cations.
7. The plant fiber product according to in any one of the preceding clauses, characterized in that said alginate is soluble in cold water.
8. The plant fiber product according to any one of the preceding clauses, characterized in that said alginate contains at least two different types of alginate with different dissolution or decomposition properties.
9. The plant fiber product according to any one of the preceding clauses, characterized in that the plant fibers are based on one or more of the following plants: tea, coffee, tobacco, cocoa, maize, herbs, yerba mate and cellulose.
10. The plant fiber product according to any one of the preceding clauses, characterized in that the plant fibers have a size distribution mainly in the range 0.1 - 0.9 mm.
11. The plant fiber product according to any one of the preceding clauses, characterized in that the plant fibers are treated with steam.
12. A method for preparing a plant fiber product for oral use, comprising the following steps:
   - mixing plant fibers with other components incorporated in the product, characterized in that the method includes the following steps:
   - admixing an alginate composition containing at least water, alginate and at least one added substance intended to be released from the product when said product is used, the composition at least in part forming an alginate matrix that retains at least a major proportion of the added substance so long as the matrix is intact, and the alginate matrix being formed so as to disintegrate and/or dissolve in the chemical and physical environment that exists in a user's mouth, and
   - mixing plant fibers with said alginate composition.
13. The method according to clause 12, characterized in that the admixing of the alginate composition is carried out with the at least one added substance being mixed with the water before the alginate is mixed in.
14. The method according to clause 12 or 13, characterized in that the plant fiber product is adapted to deliver substances to a user through mucous membranes in the user's mouth and that said at least one added substance is an active substance, for example caffeine, that can be absorbed by the mucous membranes in the mouth when said product is used.
15. The method according to in clause 14, characterized in that the at least one active substance is one or more substances belonging to any of the following categories: API (active pharmaceutical substances), food additives, natural medicaments and naturally occurring substances that can have an effect on the human body.
16. The method according to any one of clause 12 - 15, characterized in that said at least one added substance is a taste substance.

## Claims

1. A product for oral use, said product containing a mixture of at least:
- cellulose fibers;
- an alginate composition; and
- at least one added substance intended to be released from the product when said product is used,
wherein the alginate composition is distributed in the product and the alginate composition comprises at least water, alginate and the added substance, said composition containing an alginate matrix that retains at least a major proportion of the added substance so long as the matrix is intact, and the alginate matrix being formed so as to disintegrate and/or dissolve in the chemical and physical environment that exists in a user's mouth.

2. The product as claimed in claim 1, wherein the alginate composition is prepared in such a way that the alginate matrix is generated in an aqueous solution, in which solution the at least one added substance is dissolved.

3. The product as claimed in claim 1 or 2, wherein the product is adapted to deliver substances to a user through mucous membranes in the user's mouth and that said at least one added substance is an active substance that can be absorbed through the mucous membranes in the mouth when the product is used.

4. The product as claimed in claim 3, wherein the at least one active substance is one or more substances belonging to any of the following categories: API (active pharmaceutical substances), food additives, natural medicaments and naturally occurring substances that can have an effect on the human body.

5. The product as claimed in any one of the preceding claims, wherein said at least one added substance is a taste substance.

6. The product as claimed in any one of the preceding claims, wherein said alginate contains an alginate salt of monovalent cations and/or wherein said alginate completely dissolves in water at room temperature.

7. The product as claimed in any one of the preceding claims, wherein said alginate contains at least two different types of alginate with different dissolution or decomposition properties.

8. The product as claimed in any one of the preceding claims, wherein the cellulose fibers have a size distribution in the range 0.1 - 0.9 mm.

9. The product as claimed in any one of the preceding claims, wherein the cellulose fibers are treated with steam.

10. A method for preparing a product for oral use, comprising the following steps:
- mixing cellulose fibers with other components incorporated in the product, wherein the method includes the following steps:
- admixing an alginate composition containing at least water, alginate and at least one added substance intended to be released from the product when said product is used, the composition at least in part forming an alginate matrix that retains at least a major proportion of the added substance so long as the matrix is intact, and the alginate matrix being formed so as to disintegrate and/or dissolve in the chemical and physical environment that exists in a user's mouth, and
- mixing the cellulose fibers with said alginate composition.

11. The method as claimed in claim 10, wherein the admixing of the alginate composition is carried out with the at least one added substance being mixed with the water before the alginate is mixed in.

12. The method as claimed in claim 10 or 11, wherein the product is adapted to deliver substances to a user through mucous membranes in the user's mouth and that said at least one added substance is an active substance that can be absorbed by the mucous membranes in the mouth when said product is used.

13. The method as claimed in claim 12, wherein the at least one active substance is one or more substances belonging to any of the following categories: API (active pharmaceutical substances), food additives, natural medicaments and naturally occurring substances that can have an effect on the human body.

14. The method as claimed in any one of claims 10 to 13, wherein said at least one added substance is a taste substance.
